# EUROPEAN PATENT APPLICATION

(11) **EP 4 378 466 A1**
(43) Date of publication of application: **05.06.2024**
(21) Application number: 22849572.7
(22) Date of filing: 28.07.2022
(51) Int. Cl.: A61K 31/7068, A61P 35/00

(54) **PHARMACEUTICAL COMPOSITION AND ANTI-TUMOR AGENT FOR TUMORS THAT HAVE AT LEAST EITHER IMPAIRED BAP1 OR PBRM1 FUNCTION**

(30) Priority: 29.07.2021 US 202163227035 P
(71) Applicant: FUJIFILM Corporation, Tokyo 106-8620 (JP)
(72) Inventor: SAEKI Kazunori, Ashigarakami-gun, Kanagawa 258-8577 (JP); WAGES David, Scottsdale, Arizona 85259 (US); CHEUNG Kin, Wellesley, Massachusetts 02482 (US); JANKU Filip, Houston, Texas 77030 (US)
(74) Representative: Hoffmann Eitle
(86) International application number: PCT/JP2022/029062
(87) International publication number: WO 2023/008511

(57) **Abstract**

An object of the present invention is to provide a pharmaceutical composition and an anti-tumor agent that exhibit an effect on a tumor having a decreased function of at least one of BAP1 or PBRM1. According to the present invention, there is provided a pharmaceutical composition for the treatment of a tumor having a decreased function of at least one of BAP1 or PBRM1, the pharmaceutical composition including 1-(2-deoxy-2-fluoro-4-thio-β-D-arabinofuranosyl)cytosine or a salt or prodrug thereof.

## Description

### Technical Field

The present invention relates to a pharmaceutical composition and an anti-tumor agent for a tumor having a decreased function of at least one of BAP1 or PBRM1.

### Background Art

It is known that 1-(2-deoxy-2-fluoro-4-thio-β-D-arabinofuranosyl)cytosine (hereinafter, sometimes referred to as "compound A") has an excellent antitumor activity and is useful as an anti-tumor agent (Patent Document 1). In addition, salts, prodrugs, injection preparations, production methods, and uses of the compound A are also known (Patent Documents 2 to 12).

### Prior Art Documents

### Patent Documents

Patent Document 1: WO1997/038001A
Patent Document 2: WO2013/146833A
Patent Document 3: WO2011/074484A
Patent Document 4: WO2014/027658A
Patent Document 5: WO2016/068341A
Patent Document 6: WO2017/150511A
Patent Document 7: WO2018/043530A
Patent Document 8: WO2019/146129A
Patent Document 9: WO2019/146130A
Patent Document 10: WO2019/164010A
Patent Document 11: WO2019/176984A
Patent Document 12: WO2019/176985A

### SUMMARY OF THE INVENTION

Breast cancer gene 1 associated protein 1 (BAP1) has a deubiquitinating enzyme activity and is involved in DNA damage response, transcription, immune response, cell cycle, and the like. Polybromo 1 (PBRM1) is one of molecules that constitute a SWI/SNF complex that controls chromatin remodeling, and is involved in DNA damage response, cell cycle, metabolism, cell differentiation, and the like. It has been reported that BAP1 and PBRM1 exhibit a decreased function such as decreased expression or genetic mutation in various cancers. It is said that such a decreased function affects a cellular function. In addition, it has been reported that a decreased function of BAP1 or PBRM1 is associated with the development and progression of cancer.

So far, it has not been reported that the compound A specifically exhibits a therapeutic effect on a tumor having a decreased function of at least one of BAP 1 or PBRM1.

An object of the present invention is to provide a pharmaceutical composition and an anti-tumor agent that exhibit an effect on a tumor having a decreased function of at least one of BAP1 or PBRM1.

As a result of extensive studies to achieve the foregoing object, the present inventors have found that the compound A exhibits a therapeutic effect on a tumor having a decreased function of at least one of BAP1 or PBRM1. The present invention has been completed based on these findings.

That is, the present invention provides the following aspects.
(1) A pharmaceutical composition for the treatment of a tumor having a decreased function of at least one of BAP1 or PBRM1, the pharmaceutical composition including 1-(2-deoxy-2-fluoro-4-thio-β-D-arabinofuranosyl)cytosine or a salt or prodrug thereof.
(2) The pharmaceutical composition according to (1), in which the decreased function is a genetic mutation.
(3) The pharmaceutical composition according to (1) or (2), in which the tumor is a brain tumor, a hematopoietic organ tumor, a sarcoma, a thymic tumor, a liver cancer, a biliary tract cancer, a pheochromocytoma, a paraganglioma, a primitive neuroectodermal tumor, a melanoma, a kidney cancer, a mesothelioma, a uterine corpus cancer, a breast cancer, a prostate cancer, a large intestine cancer, a bladder cancer, or a thyroid cancer.
(4) The pharmaceutical composition according to (1) or (2), in which the tumor is a biliary tract cancer, a bladder cancer, a parotid cancer, or a chondrosarcoma.
(5) An anti-tumor agent for a tumor having a decreased function of at least one of BAP1 or PBRM1, the anti-tumor agent including 1-(2-deoxy-2-fluoro-4-thio-β-D-arabinofuranosyl)cytosine or a salt or prodrug thereof.
(6) The anti-tumor agent according to (5), in which the decreased function is a genetic mutation.
(7) The anti-tumor agent according to (5) or (6), in which the tumor is a brain tumor, a hematopoietic organ tumor, a sarcoma, a thymic tumor, a liver cancer, a biliary tract cancer, a pheochromocytoma, a paraganglioma, a primitive neuroectodermal tumor, a melanoma, a kidney cancer, a mesothelioma, a uterine corpus cancer, a breast cancer, a prostate cancer, a large intestine cancer, a bladder cancer, or a thyroid cancer.
(8) The anti-tumor agent according to (5) or (6), in which the tumor is a biliary tract cancer, a bladder cancer, a parotid cancer, or a chondrosarcoma.
   (A) Use of 1-(2-deoxy-2-fluoro-4-thio-β-D-arabinofuranosyl)cytosine or a salt or prodrug thereof for the production of a pharmaceutical composition for the treatment of a tumor having a decreased function of at least one of BAP1 or PBRM1.
   (B) Use of 1-(2-deoxy-2-fluoro-4-thio-β-D-arabinofuranosyl)cytosine or a salt or prodrug thereof for an anti-tumor agent for a tumor having a decreased function of at least one of BAP1 or PBRM1.
   (C) 1-(2-deoxy-2-fluoro-4-thio-β-D-arabinofuranosyl)cytosine or a salt or prodrug thereof for use in the treatment of a tumor having a decreased function of at least one of BAP1 or PBRM1.
   (D) 1-(2-deoxy-2-fluoro-4-thio-β-D-arabinofuranosyl)cytosine or a salt or prodrug thereof for use in an anti-tumor agent for a tumor having a decreased function of at least one of BAP1 or PBRM1.
   (E) A method for the treatment of a tumor having a decreased function of at least one of BAP1 or PBRM1, the method including administering an effective dose of 1-(2-deoxy-2-fluoro-4-thio-β-D-arabinofuranosyl)cytosine or a salt or prodrug thereof to a subj ect.
   (F) A method for suppressing a tumor having a decreased function of at least one of BAP1 or PBRM1, the method including administering an effective dose of 1-(2-deoxy-2-fluoro-4-thio-β-D-arabinofuranosyl)cytosine or a salt or prodrug thereof to a subj ect.
   (G) A method for the treatment of a tumor having a decreased function of at least one of BAP1 or PBRM1, the method including administering an effective dose of 1-(2-deoxy-2-fluoro-4-thio-β-D-arabinofuranosyl)cytosine or a salt or prodrug thereof to a subject, and a step of confirming the presence or absence of a decreased function such as mutation or decreased expression of at least one of BAP1 or PBRM1 in the subject prior to administering the 1-(2-deoxy-2-fluoro-4-thio-β-D-arabinofuranosyl)cytosine or the salt or prodrug thereof.

The compound A exhibits a therapeutic effect on a tumor having a decreased function of at least one of BAP1 or PBRM1. That is, according to the present invention, there are provided a pharmaceutical composition and an anti-tumor agent that are effective against a tumor having a decreased function of at least one of BAP1 or PBRM1.

### DESCRIPTION OF THE PREFERRED EMBODIMENTS

In the present invention, the range represented by "to" includes the values at both ends of "to" unless otherwise specified.

The term "subject" is a mammal such as a human, a mouse, a monkey, or a domestic livestock in need of prevention or treatment thereof, and preferably a human in need of prevention or treatment thereof.

The term "preventing" refers to inhibition of disease onset, reduction of risk of disease onset, delay of disease onset, or the like.

The term "treating" refers to amelioration of a target disease or condition, suppression (maintenance or delay) of progression of a target disease or condition, or the like.

The term "treatment" refers to preventing, treating, or the like of a variety of diseases.

The term "tumor" refers to a benign or malignant tumor.

The term "benign tumor" refers to a tumor in which a morphology of a tumor cell and a sequence of the tumor cell are similar to those of a normal cell from which such a tumor cell is derived, and which is not invasive or metastatic.

The term "malignant tumor" refers to a tumor in which a morphology of a tumor cell and a sequence of the tumor cell are different from those of a normal cell from which such a tumor cell is derived, and which is invasive or metastatic.

Hereinafter, the present invention will be described in detail.

The present invention relates to a pharmaceutical composition for the treatment of a tumor having a decreased function of at least one of BAP1 or PBRM1, which contains 1-(2-deoxy-2-fluoro-4-thio-β-D-arabinofuranosyl)cytosine (compound A) or a salt or prodrug thereof, as well as an anti-tumor agent for a tumor having a decreased function of at least one of BAP1 or PBRM1, which contains the compound A or the salt or prodrug thereof.

### (Compound A or salt or prodrug thereof)

First, the compound A or the salt or prodrug thereof will be described.

The salt may be, for example, a pharmaceutically acceptable salt, specific examples of which include a mineral acid salt, an organic carboxylate, and a sulfonate. Preferred examples of the salt include a mineral acid salt and a sulfonate.

Examples of the mineral acid salt include a hydrochloride, a hydrobromide, a hydroiodide, a nitrate, a phosphate, and a sulfate, among which a hydrochloride, a hydroiodide, a nitrate, or a sulfate is preferable, and a hydrochloride is more preferable. Examples of the organic carboxylate include a formate, an acetate, a citrate, an oxalate, a fumarate, a maleate, a succinate, a malate, a tartrate, an aspartate, a trichloroacetate, and a trifluoroacetate. Examples of the sulfonate include a methanesulfonate, a benzenesulfonate, a p-toluenesulfonate, a mesitylenesulfonate, and a naphthalenesulfonate, among which a methanesulfonate is preferable.

The salt of the compound A may be an anhydride, a hydrate, or a solvate. In a case of being simply referred to as a "salt" in the present specification, the form thereof may be an anhydride, a hydrate, or a solvate. As for the term "anhydride" used in the present specification, it refers to a state that is neither a hydrate nor a solvate, unless otherwise specified. Even in a case where it is a substance that does not originally form a hydrate or a solvate, the salt of the compound A that does not have water of crystallization, water of hydration, and an interacting solvent is included in the "anhydride" referred to in the present invention. The anhydride may also be referred to as "anhydrate". In a case where the salt of the compound A is a hydrate, the number of water of hydration is not particularly limited, and the hydrate may be a monohydrate, a dihydrate, or the like. Examples of the solvate include a methanol solvate, an ethanol solvate, a propanol solvate, and a 2-propanol solvate.

Specific examples of a particularly preferred salt of the compound A are as follows:
a methanesulfonate of 1-(2-deoxy-2-fluoro-4-thio-β-D-arabinofuranosyl)cytosine;
a hydrochloride of 1-(2-deoxy-2-fluoro-4-thio-β-D-arabinofuranosyl)cytosine; and anhydrides of any of the foregoing salts.

The prodrug refers to a compound or a salt thereof which is converted into a compound exhibiting a desired pharmacological activity, following cleavage of a functional group functioning as a prodrug by a reaction with an enzyme, a gastric juice, or the like in the body after the administration thereof.

Groups that form prodrugs include, for example, those groups described in Stella VJ et al., Prodrugs: Challenges and Rewards. Parts 1 and 2, 2007, American Association of Pharmaceutical Scientists.

The prodrug of the compound A refers to a compound or a salt thereof that converts into the compound A or a phosphate compound thereof by a reaction with an enzyme, a gastric juice, or the like under physiological conditions *in vivo.*

With regard to the prodrug of the compound A, the description of WO2016/068341A, the contents of which are incorporated herein by reference, can be incorporated and referred to.

More specifically, for example, a thionucleoside derivative represented by General Formula [1] or a salt thereof described in WO2016/068341A is incorporated herein, and a suitable one thereof is also the same as that described in WO2016/068341A. General Formula [1] described in WO2016/068341A is shown below.

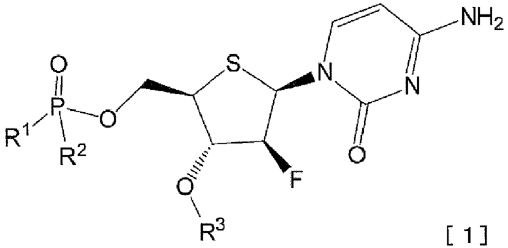

In the formula,
R¹ represents a hydroxyl group which may be protected, a C₁₋₂₀ alkoxy group which may be substituted, a C₃₋₈ cycloalkoxy group which may be substituted, an aryloxy group which may be substituted, a heterocyclic oxy group which may be substituted, or an amino group which may be substituted;
R² represents a C₁₋₂₀ alkoxy group which may be substituted, a C₃₋₈ cycloalkoxy group which may be substituted, an aryloxy group which may be substituted, a heterocyclic oxy group which may be substituted, or an amino group which may be substituted; or
R¹ and R², together with a phosphorus atom to which R¹ and R² are bonded, may form a 5- to 10-membered nitrogen/phosphorus-containing heterocyclic ring which may be substituted, a 5- to 10-membered oxygen/phosphorus-containing heterocyclic ring which may be substituted, or a 5- to 10-membered nitrogen/oxygen/phosphorus-containing heterocyclic ring; and
R³ represents a hydrogen atom; or
R² and R³, together with a phosphorus atom to which R² is bonded, an oxygen atom, methylene, two carbon atoms constituting a tetrahydrothiophene ring, and an oxygen atom to which R³ is bonded, may form a 6- to 10-membered oxygen/phosphorus-containing heterocyclic ring which may be substituted.

In the present invention, the compound A or the salt or prodrug thereof may be used alone or in combination of two or more thereof.

Next, a method for producing the compound A or the salt or prodrug thereof will be described. The compound A can be produced, for example, by the method described in WO2014/027658A. The salt of the compound A or a hydrate or solvate thereof can be produced, for example, by the method described in WO2013/146833A. The prodrug of the compound A can be produced, for example, by the method described in WO2016/068341A.

The compound A or the salt or prodrug thereof can be used as an anti-tumor agent or as an active ingredient of a pharmaceutical composition for the treatment of a tumor having a decreased function of at least one of BAP1 or PBRM1.

BAP1 is breast cancer gene 1 associated protein 1, which is a gene having a deubiquitinating enzyme activity and being involved in DNA damage response, transcription, immune response, cell cycle, and the like. With regard to BAP1, Cancer Treatment Reviews 90 (2020) 102091, the contents of which are incorporated herein by reference, can be incorporated and referred to. BAP1 is a protein consisting of 729 amino acids. The amino acid sequence of BAP1 is set forth in SEQ ID NO: 1 in the sequence listing. BAP1 has the following domains.

Ubiquitin carboxy hydrolase (UCH) domain (1-250); BARD1-binding region (182-365); HCF1 binding (HBM) domain (365-385); FoxK1/K2 binding region (477-526); BRCA1 binding region (596-721); and C-terminal binding domain (CTD) and ASXL1/2 binding domain (635-693)

PBRM1 is Polybromo 1, which is one of molecules constituting a SWI/SNF complex that controls chromatin remodeling, and is a gene involved in DNA damage response, cell cycle, metabolism, cell differentiation, and the like. With regard to PBRM1, Cancers 2020, 12, 16, the contents of which are incorporated herein by reference, can be incorporated and referred to. PBRM1 is a protein consisting of 1689 amino acids. The amino acid sequence of PBRM1 is set forth in SEQ ID NO: 2 in the sequence listing. PBRM1 has the following domains.

Bromodomain 1 (64-134); Bromodomain 2 (200-270); Bromodomain 3 (400-470); Bromodomain 4 (538-608); Bromodomain 5 (676-746); Bromodomain 6 (792-862); BAH1(Bromo-Adjacent Homology 1) domain (956-1074); and BAH2 domain (1156-1272)
SEQ ID NO: 1:
SEQ ID NO: 2:

MGSKRRRATSPSSSVSGDFDDGHHSVSTPGPSRKRRRLSNLPTVDPIAVCHEL
YNTIRDYKDEQGRLLCELFIRAPKRRNQPDYYEVVSQPIDLMKIQQKLKMEEYDDVN
LLTADFQLLFNNAKSYYKPDSPEYKAACKLWDLYLRTRNEFVQKGEADDEDDDEDG
QDNQGTVTEGSSPAYLKEILEQLLEAIVVATNPSGRLISELFQKLPSKVQYPDYYAIIKEP
IDLKTIAQRIQNGSYKSIHAMAKDIDLLAKNAKTYNEPGSQVFKDANSIKKIFYMKKA
EIEHHEMAKSSLRMRTPSNLAAARLTGPSHSKGSLGEERNPTSKYYRNKRAVQGGRLS
AITMALQYGSESEEDAALAAARYEEGESEAESITSFMDVSNPFYQLYDTVRSCRNNQG
QLIAEPFYHLPSKKKYPDYYQQIKMPISLQQIRTKLKNQEYETLDHLECDLNLMFENA
KRYNVPNSAIYKRVLKLQQVMQAKKKELARRDDIEDGDSMISSATSDTGSAKRKSKK
NIRKQRMKILFNVVLEAREPGSGRRLCDLFMVKPSKKDYPDYYKIILEPMDLKIIEHNI
RNDKYAGEEGMIEDMKLMFRNARHYNEEGSQVYNDAHILEKLLKEKRKELGPLPDD
DDMASPKLKLSRKSGISPKKSKYMTPMQQKLNEVYEAVKNYTDKRGRRLSAIFLRLP
SRSELPDYYLTIKKPMDMEKIRSHMMANKYQDIDSMVEDFVMMFNNACTYNEPESLI
YKDALVLHKVLLETRRDLEGDEDSHVPNVTLLIQELIHNLFVSVMSHQDDEGRCYSDS
LAEIPAVDPNFPNKPPLTFDIIRKNVENNRYRRLDLFQEHMFEVLERARRMNRTDSEIYE
DAVELQQFFIKIRDELCKNGEILLSPALSYTTKHLHNDVEKERKEKLPKEIEEDKLKREE
EKREAEKSEDSSGAAGLSGLHRTYSQDCSFKNSMYHVGDYVYVEPAEANLQPHIVCIE
RLWEDSAGEKWLYGCWFYRPNETFHLATRKFLEKEVFKSDYYNKVPVSKILGKCVV
MFVKEYFKLCPENFRDEDVFVCESRYSAKTKSFKKIKLWTMPISSVRFVPRDVPLPVV
RVASVFANADKGDDEKNTDNSEDSRAEDNFNLEKEKEDVPVEMSNGEPGCHYFEQL
HYNDMWLKVGDCVFIKSHGLVRPRVGRIEKVWVRDGAAYFYGPIFIHPEETEHEPTK
MFYKKEVFLSNLEETCPMTCILGKCAVLSFKDFLSCRPTEIPENDILLCESRYNESDKQ

MKKFKGLKRFSLSAKVVDDEIYYFRKPIVPQKEPSPLLEKKIQLLEAKFAELEGGDDDI
EEMGEEDSEVIEPPSLPQLQTPLASELDLMPYTPPQSTPKSAKGSAKKEGSKRKINMSG
YILFSSEMRAVIKAQHPDYSFGELSRLVGTEWRNLETAKKAEYEERAAKVAEQQERER
AAQQQQPSASPRAGTPVGALMGVVPPPTPMGMLNQQLTPVAGMMGGYPPGLPPLQG
PVDGLVSMGSMQPLHPGGPPPHHLPPGVPGLPGIPPPGVMNQGVAPMVGTPAPGGSPY
GQQVGVLGPPGQQAPPPYPGPHPAGPPVIQQPTTPMFVAPPPKTQRLLHSEAYLKYIEG
LSAESNSISKWDQTLAARRRDVHLSKEQESRLPSHWLKSKGAHTTMADALWRLRDL
MLRDTLNIRQAYNLENV

Decreased expressions or genetic mutations of BAP1 and PBRM1 have been reported in various tumors or cancers, and approximately 270 mutations in BAP1 and approximately 510 mutations in PBRM1 have been registered in The Cancer Genome Atlas, which is a cancer genome database. These mutations are said to affect cellular functions through a decrease in functions such as expression, enzyme activity, and intermolecular interaction. In addition, decreased function of BAP1 or PBRM1 has been suggested in various cancers such as a biliary tract cancer, a kidney cancer, a uveal malignant melanoma, a cutaneous malignant melanoma, a malignant mesothelioma, a uterine corpus cancer, and a breast cancer, and its relationship with cancer development and progression has been proposed.

The tumor to be treated may have genetic mutations in IDH1, FGFR2, ARID1A, BRCA1, BRCA2, SF3B1, PIK3CA, and the like in addition to BAP1 and PBRM1. These genes also have a plurality of mutation patterns. Examples of the tumor to be treated include a tumor having mutations in BAP1, IDH1 and ARID1A, a tumor having mutations in BAP1, PBRM1, FGFR2, BRCA1 and BRCA2, a tumor having mutations in BAP1 and FGFR2, and a tumor having mutations in PBRM1, IDH1, SF3B1 and PIK3CA. More specific examples of the tumor to be treated include a tumor having mutations of BAP1 deletion exons 1-3, IDH1 R132S and ARID1A duplication exons 2-15, a tumor having mutations of BAP1 deletion, PBRM1 mutation, FGFR2 truncation intron, BRCA1 S157C and BRCA2 T1679FS*3, a tumor having mutations of BAP1 M681FS*11 and FGFR2-NRAP fusion, and a tumor having mutations of PBRM1 L1617FS*8, IDH1 R132S, SF3B1 R625C and PIK3CAN1044K.

### (Pharmaceutical composition and anti-tumor agent)

The pharmaceutical composition and the anti-tumor agent according to the embodiment of the present invention may contain an additive such as an emulsifier, a surfactant, a solubilizing agent, a suspending agent, an isotonic agent, a buffering agent, a preservative, an antioxidant, a stabilizer, or an absorption promoter.

Administration routes for the pharmaceutical composition and the anti-tumor agent according to the embodiment of the present invention include intravenous, intraarterial, intrarectal, intraperitoneal, intramuscular, intratumoral, and intravesical injection methods. Administration methods include administration by syringe or drip infusion.

The daily dose of the compound A or the salt or prodrug thereof is preferably 20 mg/m² or more, more preferably 40 mg/m² to 200 mg/m², still more preferably 40 mg/m² to 150 mg/m², and even still more preferably 80 mg/m² to 150 mg/m². The dosage of the compound A or the salt or prodrug thereof can be administered as a single dose once or several divided doses.

As the administration method, a course of once-weekly dosing for 3 weeks at a single dose of 20 mg/m² or more and then cessation of medication at the 4th week can be repeated a plurality of times. In this case, the single dose is preferably 40 mg/m² to 200 mg/m², more preferably 40 mg/m² to 150 mg/m², and still more preferably 80 mg/m² to 150 mg/m².

It is preferable to confirm the presence or absence of a decreased function of at least one of BAP1 or PBRM1 in the subject before the administration of the compound A or the salt or prodrug thereof (a step of confirming a decreased expression or genetic mutation of at least one of BAP1 or PBRM1).

The method of confirming the decreased expression of at least one of BAP1 or PBRM1 may be, for example, a method of detecting mRNA, cDNA, or protein of at least one of BAP1 or PBRM1. Specific examples of the method of examining the decreased expression of at least one of BAP1 or PBRM1 include a northern blot method, a reverse transcription polymerase chain reaction (RT-PCR) method, and an immunostaining method.

Examples of the method of confirming the genetic mutation of at least one of BAP1 or PBRM1 include a method of carrying out amplification by a polymerase chain reaction (PCR) method using an allele-specific primer (and a probe) and detecting the mutation of an amplification product by means of fluorescence or luminescence; a PCR-restriction fragment length polymorphism (PCR-RFLP) method using a PCR method; a method using a DNA chip or a microarray; and a primer extension method. In addition, the mutation position to be detected may be directly sequenced.

The dosage form of the pharmaceutical composition and the anti-tumor agent according to the embodiment of the present invention may be, for example, a liquid medicinal preparation, among which an injection preparation is preferable. The dosage forms of administration can each be produced by a formulation method known to those skilled in the art.

The liquid medicinal preparation preferably contains the compound A or the salt thereof, a polyhydric alcohol having a molecular weight of 100 or less, and water.

The content of the compound A or the salt thereof in the liquid medicinal preparation is preferably 1 to 50 mg/mL, more preferably 5 to 50 mg/mL, and particularly preferably 10 to 30 mg/mL.

The polyhydric alcohol having a molecular weight of 100 or less is preferably a polyhydric alcohol having 3 or 4 carbon atoms, more preferably glycerin, propylene glycol or butanediol, and particularly preferably glycerin. Examples of the butanediol include 1,2-butanediol, 1,3-butanediol, 1,4-butanediol, and 2,3-butanediol, among which 1,3-butanediol is particularly preferable. The lower limit of the molecular weight of the polyhydric alcohol is not particularly limited, and is generally 50 or more.

The liquid medicinal preparation preferably has a pH of 1.5 to 6.9, more preferably 1.5 to 6.5, still more preferably 2.0 to 6.5, even still more preferably 2.0 to 5.0, still further more preferably 2.0 to 4.0, particularly preferably 2.6 to 3.2, and most preferably 2.8 to 3.0.

With regard to the liquid medicinal preparation, the description of WO2017/150511A, the contents of which are incorporated herein by reference, can be incorporated and referred to. The suitable composition and the suitable formulation ratio of the liquid medicinal preparation are also the same as those described in WO2017/150511A.

The pharmaceutical composition and the anti-tumor agent according to the embodiment of the present invention can be effectively used for the treatment of a tumor having a decreased function of at least one of BAP1 or PBRM1, and can be effectively used for the treatment of a tumor having a decreased function of BAP1. The decreased function refers to a mutation in at least one gene of BAP1 or PBRM1, a decreased expression of mRNA or protein generated from at least one of BAP1 or PBRM1, or the like. The tumor is preferably a malignant tumor.

The mutation in at least one gene of BAP1 or PBRM1 includes a frameshift mutation, a deletion mutation, a splice site mutation, a nonsense mutation, a missense mutation, a start codon mutation, and the like that cause a decrease in function or expression.

The pharmaceutical composition and the anti-tumor agent according to the embodiment of the present invention can be effectively used for the treatment of a tumor that has a decreased function of at least one of BAP1 or PBRM1, such as a brain tumor, a hematopoietic organ tumor, a sarcoma, a thymic tumor, a liver cancer, a biliary tract cancer, a pheochromocytoma, a paraganglioma, a primitive neuroectodermal tumor, a melanoma, a kidney cancer, a mesothelioma, a uterine corpus cancer, a breast cancer, a prostate cancer, a large intestine cancer, a bladder cancer, or a thyroid cancer. The hematopoietic organ tumor is preferably an acute myeloid leukemia, a myelodysplastic syndrome, a myeloproliferative tumor, a peripheral T-cell lymphoma, or a B-cell lymphoblastic lymphoma. The sarcoma is preferably a chondrosarcoma or an osteosarcoma. The melanoma is preferably a uveal malignant melanoma or a cutaneous malignant melanoma.

The pharmaceutical composition and the anti-tumor agent according to the embodiment of the present invention are particularly suitable for the treatment of a tumor having a decreased function of at least one of BAP1 or PBRM1, such as a biliary tract cancer, a bladder cancer, a parotid cancer, or a chondrosarcoma (preferably a chondroblastic osteosarcoma).

The target disease for the pharmaceutical composition and the anti-tumor agent according to the embodiment of the present invention is more suitably a biliary tract cancer. The biliary tract cancer is preferably a cholangiocarcinoma, and more preferably an extrahepatic cholangiocarcinoma or an intrahepatic cholangiocarcinoma.

### (Decreased function of BAP1)

Examples of the decreased function of BAP1 in the present invention include:
a decreased expression of BAP1 mRNA or protein,
a deletion mutation of glutamic acid at position 31 or a substitution of glutamic acid at position 31 with alanine, asparagine, or glycine,
a nonsense mutation of glycine at position 45 or a substitution of glycine at position 45 with alanine, arginine, or glutamic acid,
a frameshift mutation of methionine at position 681,
a substitution of arginine at position 383 with histidine,
a nonsense mutation of glutamine at position 456, a frameshift mutation of glutamine at position 456, or a substitution of glutamine at position 456 with lysine,
a nonsense mutation of serine at position 460, and
a substitution of arginine at position 700 with tryptophan or glutamine.

Above all, the pharmaceutical composition according to the embodiment of the present invention is effective for the frameshift mutation of methionine at position 681.

### (Decreased function of PBRM1)

Examples of the decreased function of PBRM1 in the present invention include:
a decreased expression of PBRM1 mRNA or protein,
a nonsense mutation of arginine at position 58 or a substitution of arginine at position 58 with glutamine,
a substitution of isoleucine at position 228 with valine,
a frameshift mutation of asparagine at position 258,
a frameshift mutation of isoleucine at position 279,
a substitution of proline at position 407 with alanine,
a nonsense mutation of arginine at position 710,
a substitution of arginine at position 876 with serine, cysteine, histidine, or leucine,
a nonsense mutation of arginine at position 1185,
a frameshift mutation of leucine at position 1617, and
a nonsense mutation of glutamine at position 1559.

Above all, the pharmaceutical composition according to the embodiment of the present invention is effective for the frameshift mutation of leucine at position 1617.

The present invention further provides:
(A) Use of 1-(2-deoxy-2-fluoro-4-thio-β-D-arabinofuranosyl)cytosine or a salt or prodrug thereof for the production of a pharmaceutical composition for the treatment of a tumor having a decreased function of at least one of BAP1 or PBRM1; and
(B) Use of 1-(2-deoxy-2-fluoro-4-thio-β-D-arabinofuranosyl)cytosine or a salt or prodrug thereof for an anti-tumor agent for a tumor having a decreased function of at least one of BAP1 or PBRM1. The details and preferred aspects of the tumor, pharmaceutical composition, anti-tumor agent, and 1-(2-deoxy-2-fluoro-4-thio-β-D-arabinofuranosyl)cytosine or salt or prodrug thereof in the above are as described above in the present specification.

The present invention further provides:
(C) 1-(2-deoxy-2-fluoro-4-thio-β-D-arabinofuranosyl)cytosine or a salt or prodrug thereof for use in the treatment of a tumor having a decreased function of at least one of BAP1 or PBRM1; and
(D) 1-(2-deoxy-2-fluoro-4-thio-β-D-arabinofuranosyl)cytosine or a salt or prodrug thereof for use in the treatment of suppressing a tumor having a decreased function of at least one of BAP1 or PBRM1. The details and preferred aspects of the tumor and 1-(2-deoxy-2-fluoro-4-thio-β-D-arabinofuranosyl)cytosine or salt or prodrug thereof in the above are as described above in the present specification.

The present invention further provides:
(E) A method for the treatment of a tumor having a decreased function of at least one of BAP1 or PBRM1, the method including administering an effective dose of 1-(2-deoxy-2-fluoro-4-thio-β-D-arabinofuranosyl)cytosine or a salt or prodrug thereof to a subj ect;
(F) A method for suppressing a tumor having a decreased function of at least one of BAP1 or PBRM1, the method including administering an effective dose of 1-(2-deoxy-2-fluoro-4-thio-β-D-arabinofuranosyl)cytosine or a salt or prodrug thereof to a subject; and
(G) A method for the treatment of a tumor having a decreased function of at least one of BAP1 or PBRM1, the method including administering an effective dose of 1-(2-deoxy-2-fluoro-4-thio-β-D-arabinofuranosyl)cytosine or a salt or prodrug thereof to a subject, and a step of confirming the presence or absence of a decreased function such as mutation or decreased expression of at least one of BAP1 or PBRM1 in the subject prior to administering the 1-(2-deoxy-2-fluoro-4-thio-β-D-arabinofuranosyl)cytosine or the salt or prodrug thereof.

The present invention further provides a method for administering an anti-tumor agent to a patient with a tumor having a decreased function of at least one of BAP1 or PBRM1, in which the anti-tumor agent includes 1-(2-deoxy-2-fluoro-4-thio-β-D-arabinofuranosyl)cytosine or a salt or prodrug thereof.

The specific dose is the same as the dose described for the pharmaceutical composition in the present specification.

The present invention further provides a method for use of 1-(2-deoxy-2-fluoro-4-thio-β-D-arabinofuranosyl)cytosine or a salt or prodrug thereof in the treatment of a tumor having a decreased function of at least one of BAP1 or PBRM1, the method including a step of administering a therapeutically effective dose of the 1-(2-deoxy-2-fluoro-4-thio-β-D-arabinofuranosyl)cytosine or the salt or prodrug thereof to a subject in need of such a treatment. The therapeutically effective dose is the same as the dose described for the pharmaceutical composition in the present specification.

The subject is preferably a human, and more preferably a tumor patient.

The subject may be a patient who has been administered gemcitabine as a pretreatment.

The subject may be a patient who has been administered gemcitabine as a pretreatment and in whom no effect greater than a partial response has been observed.

The subject may be a patient who has received a combination chemotherapy including gemcitabine as a pretreatment.

The subject may be a patient who has received a combination chemotherapy including gemcitabine as a pretreatment and in whom no effect greater than a partial response has been observed.

The subject may be a patient who has received other chemotherapy.

The subject may be a patient who is not expected to ameliorate with other chemotherapy.

According to the present invention, an ameliorating effect can be obtained even for a patient who is not expected to exhibit a therapeutic effect in the related art as described above.

The present invention further provides a method for predicting a treatment effect of administration of a pharmaceutical composition containing the compound A in a subject, the method including a step of confirming the presence or absence of a decreased function of at least one of BAP1 or PBRM1.

The present invention further provides a method for selecting a subject to whom a pharmaceutical composition containing the compound A is applied, the method including a step of confirming the presence or absence of a decreased function of at least one of BAP1 or PBRM 1.

The present invention further provides a method for determining whether or not to administer a pharmaceutical composition containing the compound A to a subject, the method including a step of confirming a decreased function of at least one of BAP1 or PBRM1.

Confirming the presence or absence of a decreased function of at least one of BAP1 or PBRM1 includes confirming decreased expression or genetic mutation of at least one of BAP1 or PBRM1. Specific examples of the method of confirming the decreased expression of at least one of BAP1 or PBRM1 and the method of confirming the genetic mutation of at least one of BAP1 or PBRM1 are as described above in the present specification.

### Examples

Hereinafter, the present invention will be described in more detail with reference to Examples, but the present invention is not limited to these Examples.

### Example 1

### <Preparation of methanesulfonate of compound A>

The methanesulfonate of 1-(2-deoxy-2-fluoro-4-thio-β-D-arabinofuranosyl)cytosine (hereinafter, also referred to as the compound A) was synthesized according to the method described in WO2013/146833A (see Example 22 described in paragraphs [0487] to [0492]) and used in the following tests.

### <Preparation of liquid pharmaceutical composition>

The methanesulfonate of the compound A was dissolved in an appropriate amount of water for injection, and the pH was adjusted using a 1 mol/L sodium hydroxide aqueous solution. An appropriate amount of water for injection was added and mixed therewith such that the concentration of the compound A was 20 mg/mL.

In addition, glycerin (manufactured by Merck & Co., Inc., molecular weight: 92) was added to a concentration of 1.5% by mass. The pH of this liquid medicinal preparation was 2.9. This liquid was filtered using a membrane filter (0.22 µm) to obtain a liquid medicinal preparation.

This liquid medicinal preparation was used in the following treatments. The treatments were carried out at The University of Texas MD Anderson Cancer Center (hereinafter, referred to as MDACC) and Sarah Cannon Research Institute (hereinafter, referred to as SCRI) in Denver, Colorado, USA.

### <Administration and determination of therapeutic effect>

A dosing cycle was repeated for cholangiocarcinoma patients, in which the compound A was administered by intravenous injection once a week from the 1st week to the 3rd week and no medication was given during the 4th week. Specifically, the compound A was administered on the 1st, 8th, and 15th days with one cycle being consisted of 28 days, and this 28-day cycle was repeated. The dose of the compound A per administration was 40 mg/m² to 135 mg/m².

The effect of the treatment was determined according to the following standards.

The subjects to be evaluated were examined by diagnostic imaging using magnetic resonance imaging (MRI), and the evaluation was made according to the following standards.
Complete response (CR): a state in which the tumor has completely disappeared
Partial response (PR): a state in which the sum of tumor sizes has decreased by 30% or more
Stable disease (SD): a state in which the size of the tumor does not change
Progressive disease (PD): a state in which the sum of tumor sizes has increased by 20% or more and the absolute value of tumor sizes has increased by 5 mm or more, or a state in which a new lesion has appeared

In addition, progression-free survival (length of time from the start of compound A administration to evaluation as PD or death) was calculated using a Kaplan-Meier method for a cholangiocarcinoma patient population in which at least one mutation was confirmed by the examination of BAP1 or PBRM1 mutations and a cholangiocarcinoma patient population in which no mutation was confirmed by the examination of BAP1 or PBRM1 mutations. A log-rank test was used to test for statistically significant differences.

### (Cholangiocarcinoma patient 1)

In one cholangiocarcinoma patient who has been administered the compound A at a dose of 40 mg/m² per administration, a cytoreductive effect (PR) of 30% or more was confirmed after 4 cycles (16 weeks). In addition, PR was confirmed even in a case where the single dose was increased to 60 mg/m² and 90 mg/m².

This patient had received, as pretreatments, a gemcitabine + cisplatin combination chemotherapy, a FOLFIRI + bevacizumab combination therapy, and an IDH inhibitor, all of which resulted in PD. In addition, this patient had a mutation in BAP1, and also had mutations in IDH1 and ARID1A.

### (Cholangiocarcinoma patient 2)

In one cholangiocarcinoma patient who has been administered the compound A at a dose of 90 mg/m² per administration, a tumor growth inhibitory effect (SD) was confirmed for a long period of 32 weeks from the start of the administration to 8 cycles. This patient had received, as pretreatments, FOLFIRINOX, a gemcitabine + cisplatin combination chemotherapy, a PEGylated recombinant IL-10 therapy, an erlotinib + bevacizumab combination therapy, and an FGFR inhibitor. The results of the respective pretreatments were SD, PR, PD, PR, and SD. In addition, this patient had mutations in both BAP1 and PBRM1, and also had mutations in FGFR2, BRCA1, and BRCA2.

### (Cholangiocarcinoma patient 3)

In one cholangiocarcinoma patient who has been administered the compound A at a dose of 135 mg/m² per administration, a growth inhibitory effect (SD) was confirmed for a long period of 48 weeks from the start of the administration to 12 cycles. The dosage was reduced to 90 mg/m² in the 3rd cycle and to 67.5 mg/m² in the 6th cycle, but SD continued. This patient had received, as pretreatments, a combination of gemcitabine and cisplatin, capecitabine, FOLFOX, ipilimumab, a PD-L1 inhibitor, a PI3K inhibitor, and sulfatinib. The results of the respective pretreatments were all SD. In addition, this patient had a mutation in BAP1 and also had a mutation in FGFR2.

### (Cholangiocarcinoma patient 4)

In one cholangiocarcinoma patient who has been administered the compound A at a dose of 90 mg/m² per administration, a growth inhibitory effect (SD) was confirmed for a long period of 56 weeks from the start of the administration to 14 cycles. The dosage was reduced to 67.5 mg/m² in the 2nd cycle, but SD continued. This patient had received gemcitabine and capecitabine as pretreatments. The results of the respective pretreatments were all PD. In addition, this patient had a mutation in PBRM1 and also had mutations in IDH1, SF3B1, and PIK3CA.

### (Cholangiocarcinoma patients 1 to 4)

Table 1 shows the age, sex, treatment facility, single dose, pretreatment (history of treatment), ECOG, therapeutic effect of the present invention, and the like of the cholangiocarcinoma patients 1 to 4. Table 2 shows the mutations of the cholangiocarcinoma patients 1 to 4 and details thereof.

**[Table 1]**

| Patient No. | Genes having mutations | Age | Sex | Treatment facility | Single dose | History of treatment | Therapeutic effect for Gem-Cis | Therapeutic effect of present invention |
|---|---|---|---|---|---|---|---|---|
| 1 | BAP1, IDH1, ARID1A | 60 | Female | MDACC | 40 mg/m² | Gem-Cis, FOLFIRI + Bevacizumab, IDH inhibitor | PD | PR |
| 2 | BAP1, PBRM1, FGFR2, BRCA1, BRCA2 | 46 | Female | MDACC | 90 mg/m² | FOLFIRINOX, Gem-Cis, PEGylated IL-10, Erlotinib + Bevacizumab, FGFR inhibitor | PD after PR | SD for long period of 32 weeks |
| 3 | BAP1, FGFR2 | 57 | Female | SCRI | 90 mg/m² | Gem-Cis, Capecitabine, FOLFOX, Ipilimumab, PD-L1 inhibitor, PI3K inhibitor | PD after SD | SD for long period of 48 weeks |
| 4 | PBRM1, IDH1, SF3B1, PIK3CA | 83 | Female | SCRI | 90 mg/m² | Gemcitabine, Capecitabine | NA | SD for long period of 56 weeks |

**[Table 2]**

| Patient No. | Genes having mutations | Details of mutations |
|---|---|---|
| 1 | BAP1, IDH1, ARID1A | BAP1 deletion exons 1-3, IDH1 R132S, ARID1A duplication exons 2-15 |
| 2 | BAP1, PBRM1, FGFR2, BRCA1, BRCA2 | BAP1 deletion, PBRM1 mutation, FGFR2 truncation intron, BRCA1 S157C, BRCA2 T1679FS*3 |
| 3 | BAP1, FGFR2 | BAP1 M681FS*11, FGFR2-NRAP fusion |
| 4 | PBRM1, IDH1, SF3B1, PIK3CA | PBRM1 L1617FS*8, IDH1 R132S, SF3B1 R625C, PIK3CA N1044K |

**[Table 3]**

| Presence or absence of genetic mutations | Median progression-free survival |
|---|---|
| BAP1/PBRM1 | 52 weeks |
| None | 16 weeks |

The patient population in which mutations were confirmed in at least one of BAP1 or PBRM1 showed a statistically significant prolongation of progression-free survival by the administration of the compound A (P value = 0.0082).

In the above table, Gem-Cis means a combination chemotherapy of gemcitabine and cisplatin. In addition, all cancer patients had received a plurality of pretreatments (history of treatment), so the details thereof were also described.

In addition, the usefulness of the present invention can also be confirmed by the following *in vitro* test.

### Example 2

### (Three-dimensional culture in vitro cell growth inhibition test using cancer cell line)

The cell line was seeded on an ultra-low adhesion plate and cultured for 4 days to form three-dimensional cell aggregates. Then, the compound A diluted to a final concentration of 1 µmol/L was added thereto, followed by further culture for 7 days, and the rate at which 1 µmol/L of the compound A inhibited cell growth was confirmed. The results are shown in Table 4.

The cell lines having BAP1 and PBRM1 mutations, specifically, NCI-H226 (a mesothelioma cell line having a BAP1 deletion mutation and a PBRM1 Q1559 nonsense mutation), and TFK1 (a biliary tract cancer cell line having a BAP1 Q456 nonsense mutation, a PBRM1 P407A mutation, and a PBRM1 splice site deletion mutation) exhibited high sensitivity to the compound A. On the other hand, the biliary tract cancer cell lines HuCCA-1 and HuH-28, which do not have a BAP1 mutation and a PBRM1 mutation, were less sensitive to the compound A.

**[Table 4]**

| Cell line | Growth inhibition rate at 1 µM of compound A | Presence or absence of genetic mutations |
|---|---|---|
| NCI-H226 | 81% | BAP1 and PBRM1 |
| TFK-1 | 57% | BAP1 and PBRM1 |
| HuCCA-1 | 4% | None |
| HuH-28 | -18% | None |

The present invention is useful as a pharmaceutical composition that exhibits a therapeutic effect on a tumor having a decreased function of at least one of BAP1 or PBRM1.

### [Sequence listing]

International application 21F00961W1JP22029062_5.xml based on International Patent Cooperation Treaty

## Claims

1. A pharmaceutical composition for the treatment of a tumor having a decreased function of at least one of BAP1 or PBRM1, the pharmaceutical composition comprising:
1-(2-deoxy-2-fluoro-4-thio-β-D-arabinofuranosyl)cytosine or a salt or prodrug thereof.

2. The pharmaceutical composition according to claim 1,
wherein the decreased function is a genetic mutation.

3. The pharmaceutical composition according to claim 1 or 2,
wherein the tumor is a brain tumor, a hematopoietic organ tumor, a sarcoma, a thymic tumor, a liver cancer, a biliary tract cancer, a pheochromocytoma, a paraganglioma, a primitive neuroectodermal tumor, a melanoma, a kidney cancer, a mesothelioma, a uterine corpus cancer, a breast cancer, a prostate cancer, a large intestine cancer, a bladder cancer, or a thyroid cancer.

4. The pharmaceutical composition according to claim 1 or 2,
wherein the tumor is a biliary tract cancer, a bladder cancer, a parotid cancer, or a chondrosarcoma.

5. An anti-tumor agent for a tumor having a decreased function of at least one of BAP1 or PBRM1, the anti-tumor agent comprising:
1-(2-deoxy-2-fluoro-4-thio-β-D-arabinofuranosyl)cytosine or a salt or prodrug thereof.

6. The anti-tumor agent according to claim 5,
wherein the decreased function is a genetic mutation.

7. The anti-tumor agent according to claim 5 or 6,
wherein the tumor is a brain tumor, a hematopoietic organ tumor, a sarcoma, a thymic tumor, a liver cancer, a biliary tract cancer, a pheochromocytoma, a paraganglioma, a primitive neuroectodermal tumor, a melanoma, a kidney cancer, a mesothelioma, a uterine corpus cancer, a breast cancer, a prostate cancer, a large intestine cancer, a bladder cancer, or a thyroid cancer.

8. The anti-tumor agent according to claim 5 or 6,
wherein the tumor is a biliary tract cancer, a bladder cancer, a parotid cancer, or a chondrosarcoma.
